# EUROPEAN PATENT SPECIFICATION

(11) **EP 3 763 822 B1**
(45) Date of publication and mention of the grant of the patent: **12.02.2025**
(21) Application number: 19761114.8
(22) Date of filing: 27.02.2019
(51) Int. Cl.: C12Q 1/68, A61P 17/00, A61K 36/00, C12Q 1/6883, C12Q 1/6851, C12Q 1/6876, A61K 36/258

(54) **USE OF MARKERS FOR IDENTIFYING EXTRACTS EFFECTIVE IN AGED SKIN CELLS**
VERWENDUNG VON MARKERN ZUR IDENTIFIZIERUNG VON EXTRAKTEN, DIE IN GEALTERTEN HAUTZELLEN WIRKSAM SIND
UTILISATION DE MARQUEURS POUR IDENTIFIER DES EXTRAITS EFFICACES DANS DES CELLULES CUTANÉES ÂGÉES

(30) Priority: 28.02.2018 CN 201810165800
(43) Date of publication of application: 13.01.2021
(73) Proprietor: Shanghai CHANDO Group Co., Ltd., 200040, Shanghai (CN)
(72) Inventor: JIANG, Dandan, Shanghai 200040 (CN); DOS SANTOS, Morgan, Shanghai 200040 (CN); LI, Hui, Shanghai 200040 (CN)
(74) Representative: Lavoix
(86) International application number: PCT/CN2019/076231
(87) International publication number: WO 2019/165963

(56) References cited:
- WO-A1-2013/117713
- CN-A- 102 625 853
- CN-A- 104 622 704
- CN-A- 105 232 600
- CN-A- 105 496 831
- US-A1- 2011 301 091
- US-B2- 9 090 937
- MUTHER CHARLOTTE ET AL: "An expression screen for aged-dependent microRNAs identifies miR-30a as a key regulator of aging features in human epidermis", AGING, vol. 9, no. 11, 19 November 2017 (2017-11-19), pages 2376 - 2396, XP055795917, DOI: 10.18632/aging.101326
- TUNDIS R ET AL: "Potential Role of Natural Compounds Against Skin Aging", CURRENT MEDICINAL CHEMISTRY, 1 January 2015 (2015-01-01), pages 1515 - 1538, XP055795920, Retrieved from the Internet <URL:https://www.eurekaselect.com/128898/article> [retrieved on 20210415]
- YASIN ZALIYATUN A.M. ET AL: "The Importance of Some Plant Extracts as Skin Anti-aging Resources: A Review", CURRENT PHARMACEUTICAL BIOTECHNOLOGY, vol. 18, no. 11, 12 February 2018 (2018-02-12), NL, pages 864 - 876, XP055795924, ISSN: 1389-2010, DOI: 10.2174/1389201019666171219105920
- JUHÁSZ MARGIT LW ET AL: "The use of natural ingredients in innovative Korean cosmeceuticals", JOURNAL OF COSMETIC DERMATOLOGY, vol. 17, no. 3, 24 January 2018 (2018-01-24), GB, pages 305 - 312, XP055795926, ISSN: 1473-2130, Retrieved from the Internet <URL:https://api.wiley.com/onlinelibrary/tdm/v1/articles/10.1111%2Fjocd.12492> DOI: 10.1111/jocd.12492

## Description

### TECHNICAL FIELD

The present invention pertains to the field of biotechnologies, and in particular, relates to a use of hsa-miR-30c-2-3p and hsa-miR-125b-1-3p for determining a snow ginseng extract to be effective or not in aged skin cells.

### BACKGROUND

In today's society, people are more and more concerned about their skin conditions, and expect to keep energetic with skin care products. Therefore, various refined products setting different skin problems are developed. With time elapsed, each consumer may face up with the problem of skin aging. Therefore, anti-aging skin care products have a dominating role in varieties of skin care products.

Skin aging is categorized into endogenous aging and exogenous aging. Endogenous aging refers to aging caused by genetic changes. Exogenous aging refers to aging caused by external factors, for example, ultraviolet irradiation, smoking, environmental pollution and the like. Although endogenous ageing is inevitable, daily use of cosmetics and skin care products could still relieve skin ageing and retard the progress of endogenous skin ageing.

With deep studies on epigenetics in recent years, people have gained new knowledge about the expression of the human genes. Various pathological and physiological conditions of the human body are not merely determined by genes. Epigenetic factors may also change the instructions for gene expression and allow the instructions to be stably inherited. The main mechanisms of epigenetics include DNA methylation, histone modification and non-coding RNAs and the like, wherein the non-coding RNAs have very important and diversified regulation functions. Therefore, much concern is given to the non-coding RNA in the molecular biology, cell biology and physiology fields, and the non-coding RNAs have a very significant value in studies. The present invention concentrates on non-coding RNAs which have been concerted in recent years and is referred to as microRNA (microRNA).

The microRNA was discovered in 1993, which is a type of endogenously small fragment RNAs, and it functions by RNA interference. The microRNA is capable of targeting messenger RNA (mRNA), and causing the mRNA degradation thereof or terminating the mRNA translation thereof. Such microRNAs thus play a very important regulation role in the cells. In addition, the microRNAs form a cluster of the greatest category of regulation molecules. The microRNAs are endogenous, and are originated from primary microRNAs (pri-miRNAs) encoded by gene groups. To date, about 700 categories of microRNAs have been authenticated. Functionality and the targets thereof are not completely interpreted or verified.

The microRNA is a small molecule commonly present in living organisms, with a length of 17 to 27 nucleotides. The microRNA, in combination with a complementary mRNA, may specifically inhibit expression of a target gene. Studies reveal that thousands of human protein encoding genes are regulated by the microRNAs, which indicates that the microRNA is a primary regulator in many important biological progresses.

The microRNA is named based on the time sequence when being discovered. Using hsa-miR-30c-2-3p as an example, "hsa" indicates that the molecule pertains to human being, "miR" indicates an identifier of the microRNA of a matured body, "30" indicates a serial number assigned according to the sequence when family members to which the microRNA pertains are discovered or when the microRNA is loaded to the public microRNA database, and "c", "-2" and '-3p" respectively indicates a precursor sequence, a gene group position and a serial number of a precursor 3' arm end that generates the matured microRNA.

At present, microRNAs of the skin are seldom studied, especially for human skin.

In animal skin, initially in mouse skin, microRNAs have been cloned. The microRNAs are very important for the establishment of the morphology of skin and coat of the animals. In recent years, it is found that the miRNAs are related to skin growth of goats and sheep. Currently, in the mouse skin, miR203 in the miRNA has been authenticated, which plays an important role in lowering cell proliferation potential in epidermal inductive differentiation. In the human body, expressions of microRNAs in the healthy skin are compared with expressions of microRNAs in skin of patients of eczema and psoriasis. MiR203 has a high level of expression in skin (relative to other organs), and is only expressed by keratinocytes. MiR203 has been proved to be a miRNA over-expressed in the skin of psoriasis patients.

At present, studies on the microRNAs of the skin concentrate on diagnosis and treatment of severe skin diseases. There are few studies concentrating on endogenous skin ageing. According to the present invention, skin samples of healthy human bodies at different ages are collected, expression profiles of microRNAs are obtained by using high-throughput human microRNA microarrays, and remarkably differential microRNA molecules are obtained by screening and induction. CN 105 496 831 discloses that a composition comprising a snow ginseng extract can promote the proliferation activity of fibroblasts from human skin to have anti-aging effects.

In analysis of action mechanisms of the remarkably differential microRNA molecules, a target gene set for the microRNA molecules may be predicted by using the Validated Target Module of the miRWalk 2.0 database. The functional and metabolic pathway enrichment analysises may be performed for the target genes set by using the bioinformatics database DAVID v6.8. The microRNA molecules playing a critical role may be obtained according to the weight of the target genes in the skin ageing function, that is, a microRNA marker of endogenous skin ageing. Finally, correlation between the microRNA marker and the predicted target genes may be verified by RT-PCR experiments.

The microRNA marker may be used to establish an in vitro biological model and test biological effects of a chemical substance according to the expression level of the microRNA marker, and the microRNA marker may be applied to the development of cosmetic active ingredients.

### SUMMARY

The present invention is intended to provide a method for developing an active substance for improving endogenous skin ageing. In this method, a microRNA marker of endogenous skin ageing is used as a test basis, such that a novel active ingredient is developed for cosmetics for improving endogenous skin ageing.

To achieve the above objective, the present invention employs the following technical solution, which is the use of hsa-miR-30c-2-3p and hsa-miR-125b-1-3p for determining a snow ginseng extract to be effective or not in aged skin cells, wherein hsa-miR-30c-2-3p is a sequence of SEQ ID NO: 1, and the hsa-miR-125b-1-3p is a sequence of SEQ ID NO: 2

A large number of skin samples of health bodies at different ages are collected, and microRNA expression profiles are tested by using high-throughput human microRNA microarrays. Differences between the microRNA expression profiles of skin at different ages are obtained through comparison, and a microRNA marker of endogenous skin ageing upon summarization is screened out.

In the microRNA marker of endogenous skin ageing, the marker is hsa-miR-30c-2-3p and hsa-miR-125b-1-3p.

The hsa-miR-30c-2-3p sequence is a sequence of SEQ ID NO.1, and the hsa-miR-125b-1-3p is a sequence of SEQ ID NO.2; wherein
SEQ ID NO.1 CUGGGAGAAGGCUGUUUACUCU
SEQ ID NO.2 ACGGGUUAGGCUCUUGGGAGCU

According to the bioinformatics analysis result, the two microRNA markers have IGF1 gene and IGFIR gene in the skin as common targets.

Through the RT-PCR test, the marker is correlated to the expressions of IGF1 gene and IGFIR gene in the skin.

IGF1 gene (Entrez ID: 3479) and IGF1R gene (Entrez ID: 3480) are respectively IGF-1 (1-type insulin-like growth factor) and IGF-1R (1-type insulin-like growth factor receptor). IGF-1 and IGF-1R have an extremely high affinity therebetween. In normal human cells, the binding of IGF-1 with IGF-1R may activate many signal pathways, for example, a longevity regulation pathway (KEGG pathway ID: hsa04211), a Fox0 signal pathway (KEGG pathway ID: hsa04068), which promote the growth and regeneration of cells.

The marker is used to develop active ingredients for cosmetics products to improve skin ageing. Specifically, 3D human skin models are reconstructed and in vitro cultivation is carried out, and the reconstructed skin models are treated by using an active substance under test. Upon completion of the cultivation, skin model samples are obtained, and semi-quantitative detection is performed for microRNA RT-PCR with the marker as the target. The active substance which obviously down-regulates (P < 0.05) the marker may be applied to develop the cosmetics for improving skin ageing.

As compared with the prior art, the present invention has the following notable advantages:

According to the present invention, a joint evaluation is carried out for the microRNA microarray technology and the expression of the microRNAs of the endogenously aged skin, and microRNA markers of the endogenous skin ageing is screened out. The effect detection method with the active substance with the marker as the target has the advantages of high efficiency, high specificity and high sensitivity, and is suitable for developing the cosmetics ingredients for improving endogenous skin ageing.

Specifically, the present invention provides a method for screening endogenous skin ageing targets. The method includes the following steps:
step 1: collecting a plurality of skin samples, and grouping the samples based on donor age;
step 2: extracting total RNAs from the plurality of skin samples;
step 3: hybridizing the total RNA samples with human microRNA microarrays;
step 4: scanning the microarrays, and processing data to obtain microRNA expression profiles;
step 5: processing the data to obtain the list of differential microRNAs;
step 6: performing functional and metabolic pathway enrichment analysises for a target gene set for the differential microRNAs.

Preferably, the present invention is performed in the sequence of step 1 to 6. However, a person skilled in the art would find it obvious to adjust the sequence of the steps according to the actual needs, and a technical means similar to the present invention is used to implement the same functions and achieve the same effects.

In one or more specific embodiments of the present invention, the differential microRNA is a marker which is a microRNA or microRNAs targeting an IGFI gene and/or IGFIR gene in skin cells.

The differential microRNA is hsa-miR-30c-2-3p and hsa-miR-125b-1-3p in the skin cells. In one or more specific embodiments of the present invention, the skin cells are selected from one or more of dermal fibroblasts and epidermal keratinocytes.

In one or more specific embodiments of the present invention, the skin cells are selected from one or more of dermal fibroblasts and epidermal keratinocytes. In one or more specific embodiments of the present invention, the differential microRNA is marked by a microRNA or microRNAs with IGFI gene and/or IGFIR gene as a common targetThe differential microRNA is hsa-miR-30c-2-3p and hsa-miR-125b-1-3p in skin cells.

In one or more specific embodiments of the present invention, regulation over the target of the skin cells by the active substance is tested by RT-PCR method. Preferably, in the RT-PCR method, an expression level of the target in the skin cells is measured by 2^{-ΔCt}. More preferably, a measurement standard of a test result by the RT-PCR method is: whether an expression level of the target of the skin cells subjected to treatment by the active substance is remarkably reduced relative to a control group (P < 0.05).

In one or more specific embodiments of the present invention, the active substance is applied as an external skin preparation. Preferably, the external skin preparation is selected from the group consisting of one or more of a face care product, a makeup product, a hair care product and a body care product.

The present invention provides an active substance for improving endogenous skin ageing, wherein by comparing a sample subjected to treatment by the active substance with a sample in a control group not subjected to the treatment by the active substance, hsa-miR-30c-2-3p and hsa-miR-125b-1-3p selected from skin cells is used as a target, wherein an expression level of the target is measured by 2^{-ΔCt} by RT-PCR method, and the expression level the sample subjected to the treatment by the active substance is remarkably reduced relative to the control group (P < 0.05). The active substance is a snow ginseng extract containing composition.

### BRIEF DESCRIPTION OF THE DRAWINGS

FIG. 1 illustrates the cluster heat map of differential microRNAs between young skin samples and aged skin samples screened out by high-throughput microarrays;
FIG. 2 illustrates the RT-PCR test results of IGF1 gene in the young skin samples and the aged skin samples ("*" indicates that P < 0.05);
FIG. 3 illustrates the RT-PCR test results of IGF1R gene in the young skin samples and the aged skin samples ("*" indicates that P < 0.05);
FIG. 4 illustrates the RT-PCR test results of SOD2 gene in the young skin samples and the aged skin samples ("*" indicates that P < 0.05);
FIG. 5 illustrates the differential expression levels of the microRNA marker of endogenous skin aging in 3D reconstructed skin model in a group subjected to treatment by an active substance and a control group not subjected to treatment by the active substance ("*" indicates that P < 0.05, and "**" indicates that P < 0.01); and
FIG. 6 illustrates the histological staining results of the 3D skin model in the group subjected to the treatment by the active substance and the non-treatment control group.

### DETAILED DESCRIPTION

For better understanding of the present invention, hereinafter the present invention is further described with reference to some embodiments and accompanying drawings. Experimental methods with specific conditions unstated in the following embodiments are routine methods with customary conditions that are readily known by a person skilled in the art, or carried out in accordance with the specifications of relevant products.

In one or more of the following examples, an active substance used is a snow ginseng extract containing 0.02% of snow ginseng extract and 99.8% of glacial water. The snow ginseng from Yunnan is ground into coarse powder with a particle size of 40 to 100 meshes; 8 to 12 times of volume of 50 to 90% of ethanol is added; ultrasonic extraction is carried out for two times each of which lasts 2 hours; filtrates are combined, condensed to 150 mL, enriched via an AB-8 macroporous absorptive resin, and eluted with water, 30% ethanol and 70% ethanol sequentially; and a part eluted with the 70% ethanol is collected, and concentrated to dryness to obtain a snow ginseng extract. The glacial water is originated from the Dorji Qudengnima Spring in the Himalayas (5128 meters above the sea level); each liter of the glacial water contains the following components: Sr⁺ 0.2-0.5 mg, K⁺ 0.5-10.0 mg, Na⁺ 1.0-5.0 mg, Ca²⁺ 14.0-30.0 mg and Mg²⁺ 2.0-10.0 mg; a total content of a soluble solid is 60 to 150 mg/L; a pH value of the glacial water is 7.4; and a deuterium content in the glacial water is 132 ppm. A composition containing snow ginseng extract is prepared by mixing the snow ginseng extract with the glacial water.

### Example 1: Testing on the microRNA expression profiles of young skin samples and aged skin samples using high-throughput microarrays

### I. Acquisition of skin samples

Fresh skin samples were purchased from Shanghai Outdo Biotech Co., Ltd.. The donors were healthy females aged in the range between 30 to 40 years old, and were born and resident in Shanghai, and totally 8 samples were collected. This group had an average age of 33.5±2.6. Donors of skin samples were healthy females those were over 50 years old and born and resident in Shanghai, and totally 7 samples were collected. This group had an average age of 62.0±9.7.

Specifically, skin tissues were collected upon the orthopaedic surgery, and the skin tissues were treated as soon as possible. The skin tissues were sheared, with a thickness not exceeding 0.5 cm, into small tissue blocks with a dimension of 0.5 cm × 1 cm × 1 cm (thickness × length × width); the small tissue blocks were placed into sterile Cryo tubes; 2 ml of RANlater (Sigma) was added to each tube to immerse the tissue for storage; and one skin tissue block was placed into one Cryo tube and the Cryo tube was stored in a low-temperature refrigerator at -80°C.

### II. Total RNA extraction from the skin samples

Ordinary tissues for use in extraction, and mirVana^{™} miRNA Isolation Kit without phenol of the cell miRNA were used, and total RNA extracted was carried out for the samples in accordance with the standard operation procedures specified by the manufacturers. The total RNA obtained through extraction was subjected to quality inspection by Agilent Bioanalyzer 2100 (from Agilent Technologies) and the qualified total RNA was set aside as a reserve.

### III. Test on high-throughput microRNA chip Expression profiles

An Agilent Human miRNA microarray covering 2549 human-related microRNAs was used, and the database used was miRBase V21.0. The total RNA samples were subjected to dephosphorylation, denaturation and ligation, and then microarray hybridization. Afterwards, the samples were washed, the chip was scanned and data was read and normalized to obtain the microRNA expression profiles of the young skin samples and the aged skin samples.

### Example 2: Screening of differential microRNAs between young skin samples and aged skin samples

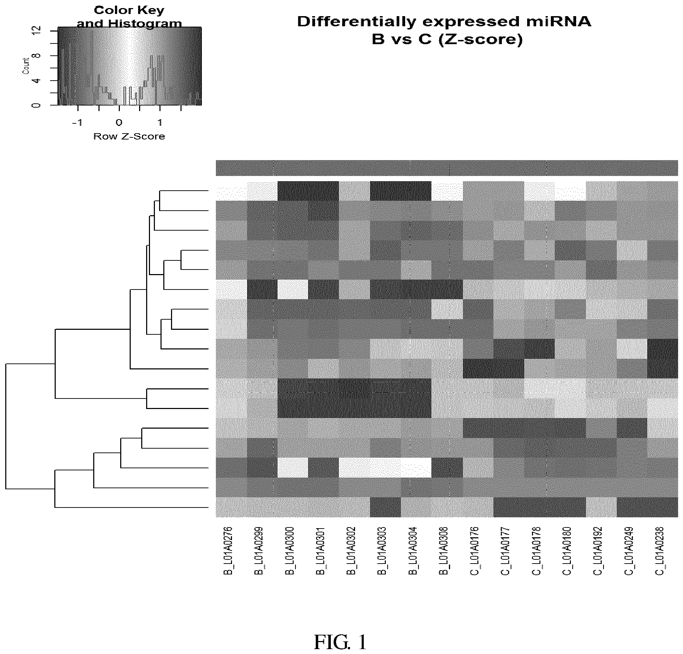

Differential expressions of the microRNAs in the young skin samples and the aged skin samples were studied by using an AgiMicroRna R analysis toolkit, and the analysis toolkit processed test data based on a limma linear model. The cluster heat map in FIG. 1 showed the analysis result. In 17 microRNA molecules, the expression levels of the young skin samples were obviously different from those of the aged skin samples. In aged skin samples, 12 microRNA molecules had obviously increasedexpression levels, and 5 microRNA molecules had obviously decreasedexpression levels. Differences of the expressions of these microRNA molecules were as listed in Table 1.

**Table 1 List of microRNA molecules showing significant differences in the young skin samples and the aged skin samples screened out by the microarray experiment (for reference and comparison, the microRNA molecules are marked by hsa-miR-a, b, c, ...m, n, o).**

| microRNA molecule | Expression level in young skin samples | Expression level in aged skin samples | Fold change | P value |
|---|---|---|---|---|
| hsa-miR-a | 106.776 | 0.680 | -156.95 | 3.25E-03 |
| hsa-miR-b | 729.352 | 6.532 | -111.66 | 6.65E-03 |
| hsa-miR-c | 120.569 | 2.005 | -60.13 | 5.47E-04 |
| hsa-miR-d | 40.286 | 2.615 | -15.40 | 8.60E-03 |
| hsa-miR-e | 9.882 | 1.334 | -7.41 | 6.81E-03 |
| hsa-miR-f | 4.060 | 21.219 | 5.23 | 6.43E-03 |
| hsa-miR-g | 1.509 | 8.155 | 5.41 | 4.73E-03 |
| hsa-miR-h | 1.788 | 10.045 | 5.62 | 3.12E-03 |
| hsa-miR-i | 3.449 | 19.396 | 5.62 | 7.15E-03 |
| hsa-miR-j | 0.899 | 5.326 | 5.92 | 1.12E-03 |
| hsa-miR-k | 1.657 | 9.950 | 6.00 | 4.78E-03 |
| hsa-miR-1 | 2.146 | 14.161 | 6.60 | 6.11E-03 |
| hsa-miR-m | 2.743 | 24.789 | 9.04 | 2.90E-03 |
| hsa-miR-n | 1.414 | 13.330 | 9.42 | 5.84E-03 |
| hsa-miR-o | 2.182 | 43.229 | 19.81 | 4.45E-05 |
| hsa-miR-125b-1-3p | 4.620 | 99.998 | 21.65 | 5.48E-03 |
| hsa-miR-30c-2-3p | 24.761 | 690.818 | 27.90 | 5.19E-03 |

### Example 3: microRNA markers and target genes of endogenous skin ageing obtained via bioinformatics analysis

The target genes of the differential microRNAs of endogenous skin ageing were predicted by using the Validated Target Module of the miRWalk 2.0 database, and a group of predicted gene sets were generated.

Functional and metabolic pathway enrichment analysises was carried out by using the bioinformatics database DAVID v6.8. The enrichment analysis was based on five annotation category modules, including the gene body (GO_TERM), KEGG pathway, BIOCARTA pathway, InterPRO database and UP_KEYWORDS database. Table 2 listed part of the analysis results, which includes the five most significant functionality or metabolism pathway annotations.

**Table 2 Functional and metabolic pathway enrichment analysises results of the target genes of the differential microRNAs.**

| Enrichment analysis annotation module | Functionality or metabolism pathway annotation | Relevant genes |
|---|---|---|
| KEGG_PATHWAY | hsa04068:FoxO signaling pathway | IGF1, IGF1R, SOD2, S1PR1 |
| UP_KEYWORDS | Mitochondrion | NR3C1, PRNP, SOD2, COX20 |
| GOTERM_BP_DIRECT | GO:0043066-negative regulation of apoptotic process | PRNP, IGF1, IGF1R, SOD2 |
| BIOCARTA | h_longevity Pathway: The IGF-1 Receptor and Longevity | IGF1, IGF1R, SOD2 |
| GOTERM_MF_DIRECT | GO:0031625-ubiquitin protein ligase binding | CDKN1A, HSPA1B, NLK, SCAMP3, ELOVL1 |

In the functionality and metabolism pathways listed in Table 2, the IGF1 gene, IGFIR gene and SOD2 gene occupy a greater proportion, and Fox0 and longevity pathways to which these genes belong are related to the growth and regeneration process of the cells. Therefore, it is speculated that hsa-miR-30c-2-3p and hsa-miR-125b-1-3p of the microRNA molecules regulating the IGF1 gene, IGF1R gene and SOD2 gene are markers of endogenous skin ageing.

### Example 4: Target genes verified and predicted by RT-PCR experiments

### I. Primer design

The preparation method of the skin total RNA was the same as the process in Example 1. By RT-PCR experiment, differential expression levels of the IGF1 gene, IGF1R gene and SOD2 gene in the young skin samples and aged skin samples were verified. In this example, 18S was used as an internal reference gene. The primer sequences of the genes under test and the internal reference gene were as follows:
IGF1-F: 5' AACACACTGCAGGAGGGACT 3' (SEQ ID NO.3)
IGF1-R: 5' GCTGCGTGATATTTGAAAGG 3' (SEQ ID NO.4)
IGF1R-F: 5' GGTTGAGGTGAGAGGTTTGC 3' (SEQ ID NO.5)
IGF1R-R: 5' ATTATTTGCGGTGCATCCAT 3' (SEQ ID NO.6)
SOD2-F: 5' CCAGGGGAAGTACTGTTTGG 3' (SEQ ID NO.7)
SOD2-R: 5' TCTTGCTGGGATCATTAGGG 3' (SEQ ID NO.8)
18S-F: 5' TCTGTGATGCCCTTAGATGTCC 3' (SEQ ID NO.9)
18S-R: 5'AATGGGGTTCAACGGGTTAC 3' (SEQ ID NO.10)

Upon completion of primer design, non-specificity of sequences of the amplified target fragments was prevented by BLAST analysis (https://blast.ncbi.nlm.nih.gov/Blast.cgi) . The primers were synthesized by Shanghai Sangon Biotech Co., Ltd.

### II. Reverse transcription

The RNA samples were taken out from a -80°C refrigerator and thawed at 4°C. The reaction solution was formulated in a 0.2 ml PCR tube, and afterwards, the PCR tube was placed at 37°C for incubation for 15 min, denatured at 98°C for 5 min, and then kept at 4°C.

### III. SYBR Green qPCR

The PCR tube was placed in a PCR instrument for reaction, incubated at 50°C for 2 min and then at 95°C for 10 min. 40 cycles were carried out by this way. Afterwards, 95°C for 15s and 60°C for 1 min, and finally a dissolution curve was added.

### IV. Data processing

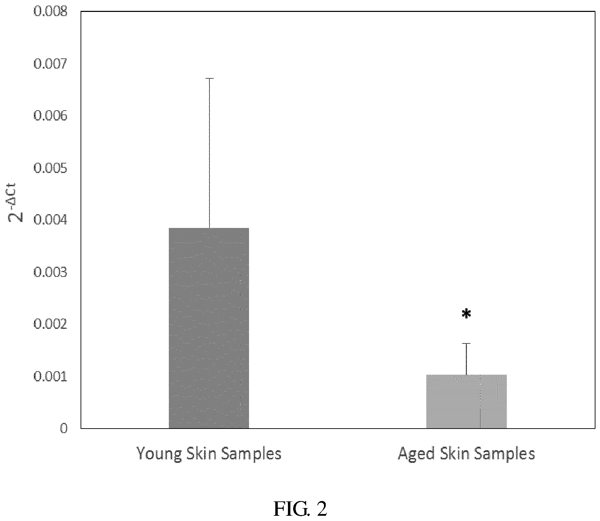

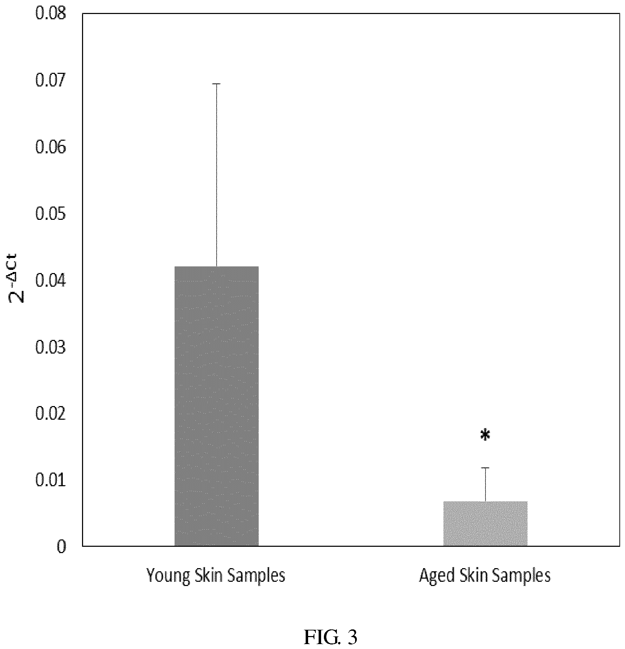

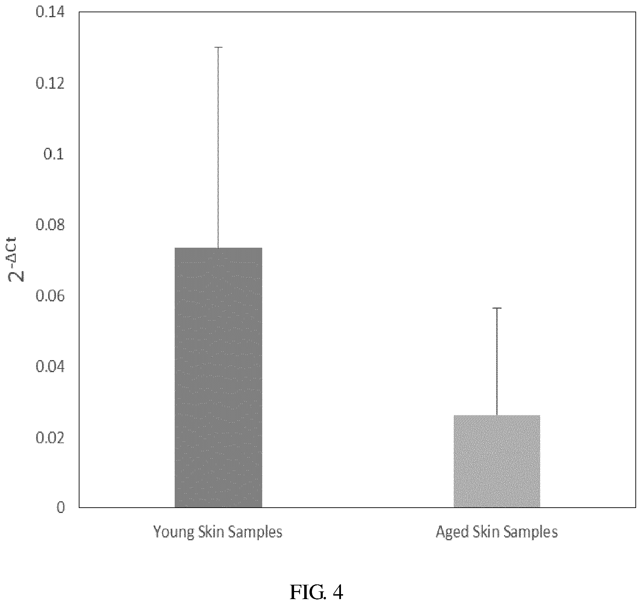

2^{-ΔCt} values obtained by comparing the expression levels of the target genes with the internal reference gene were measured, and data was as listed in Table 3. FIG. 2 illustrated the comparison between the young skin and the aged skin, and as illustrated in FIG. 2, the expression level of the IGF1 gene in the aged skin samples was remarkably lowered (P < 0.05) than the IGF1 gene in the young skin samples. As illustrated in FIG. 3, the expression level of the IGF1R gene in the aged skin samples was also significantly lowered (P < 0.05) than the IGF1R gene in the young skin samples. This result was in accordance with the variation trend of the difference of microRNA markers subjected to endogenous skin ageing. It revealed that the microRNA markers subjected to endogenous skin ageing are correlated to the expression of IGF1 gene and IGFIR gene. As illustrated in FIG. 4, there was no significant difference between the expression levels of the SOD2 gene in young and aged skin samples.

**Table 3 Evaluation results of the expression levels of the target genes in the young skin samples and the aged skin samples**

| Target gene under test | Expression level in young skin samples (2^{-ΔCt}) | Expression level in aged skin samples (2^{-ΔCt}) | P value |
|---|---|---|---|
| IGF1 | (3.85±0.29)×10-3 | (1.03±0.59)×10-3 | 0.0376 |
| IGF1R | (4.20±2.75×10-2 | (6.85±5.03)×10-3 | 0.0105 |
| SOD2 | (7.34±5.67)×10-2 | (2.65±2.98)×10-2 | 0.0965 |

### Example 5: Efficacy evaluation of the cosmetic active substances targeting microRNA markers subjected to endogenous skin ageing

### I. Extraction of aged skin cells

By using purchased fresh facial skin samples from a 52-year-old female donor, primary cell isolation was carried out for dermal fibroblasts and epidermal keratinocytes in a sterile environment. The skin was sterilized with iodide and 75% alcohol, and washed with PBS. Subcutaneous adipose tissues and vessels were trimmed with scissors The skin was cut into small blocks with a dimension of 0.5 cm × 0.5 cm and digested at 4°C for 15 hours with Dispase II (from Roche). The epidermal layer and the dermal layer were isolated. The dermal layer was digested with 0.05% trypsin (from Invitrogen) for 13 minutes and centrifuged for 10 minutes at a rotation speed of 1000 rpm, the supernatant was discarded, and the cells were resuspended with a K-FSM (from Invitrogen) and then seeded into a 162 cm² flaskand placed in a cell incubator at 37°C, 5% CO₂, and saturated humidity for cultivation. When the cell layer grew until 70% to 80% confluent, the cells were collected and frozen to establish a dermal fibroblast cell bank. The epidermal layer was digested with collagenase A (from Roche) for 4 hours and centrifuged for 10 minutes at a rotation speed of 1000 rpm, the supernatant was discarded, the cells were resuspeded with DMEM (from Invitrogen) containing 10% FBS, and the primary cultivation was carried out for the cells and the cells were frozen to establish an epidermal keratinocyte bank.

### II. Effects of the active substance on the 3D reconstructed aged skin model

The dermal fibroblasts and the epidermal keratinocytes were subjected to in vitro expansion and seeded onto a biocompatible scaffold material for 3D cultivation, and 6 reconstructed aging skin models were established. The cells were cultivated in groups 3 days after the seeding, 3 skin models in the control group were cultivated by using a common cultivation medium, and the cells in the treatment group were cultivated by using a common cultivation medium with composition containing snow ginseng extract. The 3D cultivation was stopped till the 42^{th} day.

### III. Test on expression of the microRNA marker subjected to endogenous ageing by RT-PCR

The method is the same as storage of the skin samples and extraction of the total RNA. The microRNA reverse transcription primers were directly purchased from ABI. The total RNA of the skin samples was reverse transcribed into cDNA, and then RT-PCR reaction was carried out by ReverTraAce qPCR Kit (from TOYOBO) to test the expression level of the marker, wherein U6 was used as an internal reference. The reaction system is operated in accordance with an instruction manual of a Power SYBR Green PCR Master Mix (from ABI), each skin sample was loaded in triplicate to a 384 well-plate for cultivation. The reaction conditions were as follows: reaction for 15s at 95°C, reaction for 1 min at 60°C, and 40 cycles. The expression level of the microRNA was tested by 2^{-ΔCt} obtained by comparison with the internal control U6.

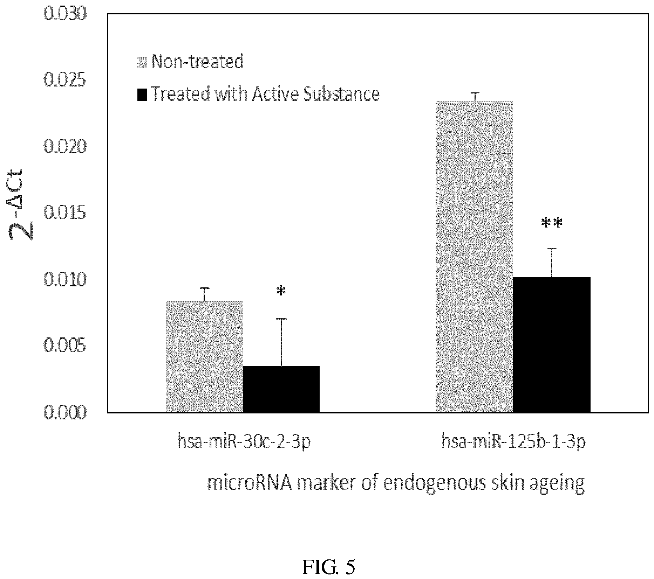

FIG. 5 illustrates the results. As illustrated in FIG. 5, the expression levels of hsa-miR-30c-2-3p and hsa-miR-125b-1-3p in the aged reconstructed skin subjected to treatment by the snow ginseng extract were obviously lowered (*P < 0.05, **P < 0.01) relative to the control group, which revealed that the snow ginseng extract is capable of regulating the microRNA marker of endogenous skin ageing. The specific data was as listed in Table 4.

**Table 4 Impacts of the active substance on the microRNA marker of endogenous skin ageing in the reconstructed skin model**

| Sample group | Control group | Active substance treated group | P value |
|---|---|---|---|
| Expression level of hsa-miR-30c-2-3p (2^{-ΔCt}) | (8.387±0.938) × 10⁻³ | (3.430±0.570) × 10⁻³ | 0.0235 |
| Expression level of hsa-miR-125b-1-3p (2^{-ΔCt}) | (2.344±0.359) × 10⁻² | (1.020±0.215) × 10⁻² | 0.00946 |

### IV. Impacts of the active substance on the tissue structure of the aged reconstructed skin model

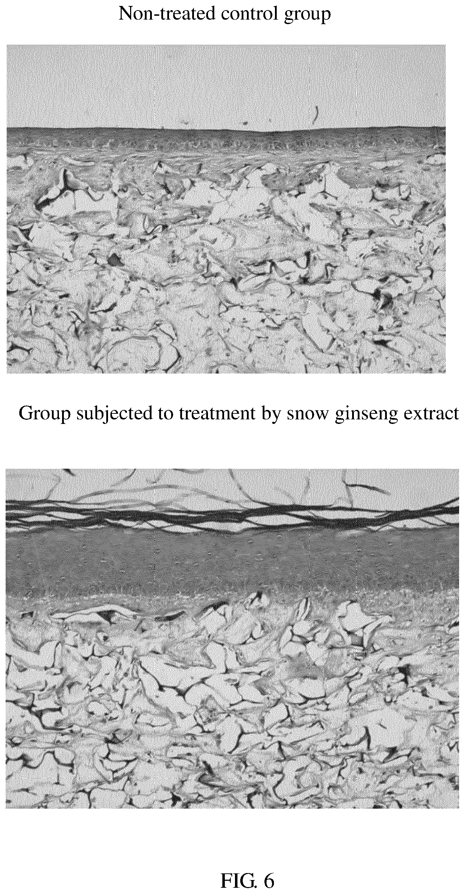

In the established aged reconstructed skin model in the above example, on the 42^{th} day when the 3D cultivation was stopped, the skin models in the group subjected to treatment by the snow ginseng extract and in a control group not subjected to treatment by the ginseng extract were collected and placed to a 10% formalin solution for fixation and paraffin embedding, and then cut into thin slices having a thickness of 5 µm. The slices were histochemically stained by using Masson's Trichrome, and microscopic images were taken by using a microscope. FIG. 6 illustrated the result, which revealed that the entire tissue structure of the non-treatment reconstructed aging skin model was poor, whereas the reconstructed aging skin model subjected to treatment by the active substance exhibited a complete tissue structure and had a well-organized stratum basale of epidermis, an adhesive layer having an improved thickness, and a well differentiated stratum corneum. In this experiment, it may be apparently seen from the histochemical staining result that the snow ginseng extract improves the aged skin. The histochemical staining result, in combination with the above RT-PCR result obtained by testing the microRNA marker of endogenous skin ageing, reveals that the results of these two test methods are correlated, and the effect of the snow ginseng extract in improving endogenous skin ageing may be verified by testing the microRNA marker of the endogenous skin ageing.

Described above are merely some exemplary examples for illustrating the present invention.

## Claims

1. A use of hsa-miR-30c-2-3p and hsa-miR-125b-1-3p for determining a snow ginseng extract to be effective or not in aged skin cells, wherein hsa-miR-30c-2-3p is a sequence of SEQ ID NO: 1, and the hsa-miR-125b-1-3p is a sequence of SEQ ID NO: 2.

2. The use according to claim 1, wherein the snow ginseng extract is prepared by:
grounding a snow ginseng into coarse powder with a particle size of 40 to 100 meshes;
adding 8 to 12 times of volume of 50 to 90% of ethanol,
carrying out ultrasonic extraction for two times each of which lasts 2 hours;
combining and condensing filtrates to 150 mL,
enriching via an AB-8 macroporous absorptive resin,
eluting with water, 30% ethanol and 70% ethanol sequentially;
Collecting a part eluted with the 70% ethanol, and
concentrating to dryness to obtain the snow ginseng extract.

3. The use according to claim 2, wherein the glacial water comprises Sr⁺ 0.2-0.5 mg/L, K⁺ 0.5-10.0 mg/L, Na⁺ 1.0-5.0 mg/L, Ca²⁺ 14.0-30.0 mg/L and Mg²⁺ 2.0-10.0 mg/L, and has a total content of a soluble solid of 60 to 150 mg/L, a pH value of the glacial water of 7.4, and a deuterium content in the glacial water of 132 ppm.

## Patentansprüche

1. Verwendung von hsa-miR-30c-2-3p und hsa-miR-125b-1-3p zum Bestimmen, ob ein Snow-Ginseng-Extrakt in gealterten Hautzellen wirksam ist, wobei hsa-miR-30c-2-3p eine Sequenz von SEQ ID NO: 1 ist, und das hsa-miR-125b-1-3p eine Sequenz von SEQ ID NO: 2 ist.

2. Verwendung nach Anspruch 1, wobei der Snow-Ginseng-Extrakt hergestellt wird durch:
Zerkleinern eines Snow-Ginsengs zu grobem Pulver mit einer Teilchengröße von 40 bis 100 Mesh;
Hinzufügen des 8- bis 12-fachen Volumens von 50 bis 90 % Ethanol,
Durchführen einer Ultraschallextraktion jeweils zwei Mal, wovon jede 2 Stunden dauert;
Kombinieren und Kondensieren der Filtrate auf 150 ml,
Anreichern über ein makroporöses Absorptionsharz AB-8,
Eluieren mit Wasser, 30 %igem Ethanol und 70 %igem Ethanol nacheinander;
Auffangen eines Teils, der mit 70 %igem Ethanol eluiert wurde, und Konzentrieren bis zur Trockenheit, um den Snow-Ginseng-Extrakt zu erlangen.

3. Verwendung nach Anspruch 2, wobei das Gletscherwasser Sr⁺ 0,2-0,5 mg/L, K⁺ 0,5-10,0 mg/L, Na⁺ 1,0-5,0 mg/L, Ca²⁺ 14,0-30,0 mg/L und Mg²⁺ 2,0-10,0 mg/L umfasst und einen Gesamtgehalt an löslichem Feststoff von 60 bis 150 mg/L, einen pH-Wert des Gletscherwassers von 7,4 und einen Deuteriumgehalt im Gletscherwasser von 132 ppm aufweist.

## Revendications

1. Utilisation de hsa-miR-30c-2-3p et hsa-miR-125b-1-3p pour déterminer si un extrait de ginseng des neiges est efficace ou non dans les cellules de la peau âgée, dans laquelle hsa-miR-30c-2-3p est une séquence de SEQ ID NO : 1, et le hsa-miR-125b-1-3p est une séquence de SEQ ID NO : 2.

2. Utilisation selon la revendication 1, dans laquelle l'extrait de ginseng des neiges est préparé par :
broyage d'un ginseng des neiges en poudre grossière avec une taille de particules de 40 à 100 mesh ;
ajout de 8 à 12 fois le volume de 50 à 90 % d'éthanol, extraction par ultrasons deux fois, chacune d'une durée de 2 heures ;
combinaison et condensation de filtrats à 150 ml,
enrichissement par l'intermédiaire d'une résine absorbante macroporeuse AB-8, élution avec de l'eau, 30 % d'éthanol et 70 % d'éthanol séquentiellement ;
collecte d'une partie éluée avec l'éthanol à 70 %, et
concentration jusqu'à siccité pour obtenir l'extrait de ginseng des neiges.

3. Utilisation selon la revendication 2, dans laquelle l'eau glacée comprend Sr⁺ 0,2-0,5 mg/l, K⁺0,5-10,0 mg/l, Na⁺ 1,0-5,0 mg/l, Ca²⁺ 14,0-30,0 mg/l et Mg²⁺ 2,0-10,0 mg/l, et a une teneur totale en solide soluble de 60 à 150 mg/l, une valeur de pH de l'eau glacée de 7,4, et une teneur en deutérium dans l'eau glacée de 132 ppm.
